# EUROPEAN PATENT SPECIFICATION

(11) **EP 1 253 945 B1**
(45) Date of publication and mention of the grant of the patent: **04.10.2006**
(21) Application number: 00980880.9
(22) Date of filing: 30.11.2000
(51) Int. Cl.: A61K 45/06, A61K 31/55, A61K 38/55, A61P 9/00

(54) **ACE INHIBITOR-VASOPRESSIN ANTAGONIST COMBINATIONS**
ACE INHIBITOR-VASOPRESSIN ANTAGONIST KOMBINATIONEN
COMBINAISONS D'INHIBITEURS D'ACE ET D'ANTAGONISTES DE VASOPRESSINE

(30) Priority: 26.01.2000 US 178169 P
(43) Date of publication of application: 06.11.2002
(73) Proprietor: Astellas Pharma Inc., Tokyo 103-8411 (JP)
(72) Inventor: PRESSLER, Millton, Lethan, Saline, MI 48176 (US)
(74) Representative: Bates, Philip Ian
(86) International application number: PCT/US2000/032569
(87) International publication number: WO 2001/054677

(56) References cited:
- EP-A- 0 205 334
- EP-A- 0 229 329
- EP-A- 0 344 995
- EP-A- 0 365 134

## Description

### FIELD OF THE INVENTION

This invention relates to compositions comprising a compound which inhibits the actions of angiotensin-converting enzyme and a compound which inhibits the actions of vasopressin enzymes, and the use of such compositions for the manufacturing of pharmaceuticals for treating ventricular dilation, heart failure, and cardiovascular pathologies.

### BACKGROUND OF THE INVENTION

Heart failure is a pathophysiologic state in which the heart is unable to pump sufficient blood to meet the metabolic needs of the body. It may be caused by a number of factors affecting the myocardium, some altering systolic function and others interfering with diastolic function and/or both. Ischemic heart disease is the most common cause of heart failure in Western countries. Other common etiologies include: (1) hypertension and hypertrophic cardiomyopathy; (2) dilated cardiomyopathy of known cause (e.g., secondary to diabetes; hypo- or hyperthyroidism; viral or parasitic infection); (3) idiopathic dilated cardiomyopathy; and (4) congenital or acquired valvular disease. Severity of chronic heart failure (CHF) is often categorized by the New York Heart Association (NYHA) Functional Classification system.

Development and progression of CHF is a major unsolved problem. Heart failure is one of the few cardiovascular diseases with increasing prevalence, now afflicting 3 to 4 million persons in the United States of America (USA), a like number in Europe, and 200,000 in Canada. It accounts for tens of billions of dollars of health care expenditure in the USA alone. It is more common with advancing age: 75% of hospitalized CHF patients are over 65 and 50% are over 75 years of age. CHF admissions comprise the No. 1 diagnosis-related group (DRG) for the Medicare population; 800,000 to 900,000 hospitalizations in the USA per year are related to CHF decompensation. The prognosis remains poor despite increasing understanding of mechanisms and new treatments. Around 465,000 new cases of heart failure develop in the USA annually and there are over 250,000 deaths. Fifty percent to 60% of patients are dead within 5 years of diagnosis; 1-year mortality is approximately 40% to 50% for those with severe functional impairment Roughly 20% of the heart failure population (600,000 persons in the USA) suffers from severe (NYHA Functional Class III/IV) CHF.

Chronic treatments for CHF include digoxin, diuretics, angiotensin converting enzyme (ACE) inhibitors, the combination of hydralazine and isosorbide dinitrate, and β-blockers, specifically carvedilol. Acute medical therapies for heart failure also include inotropic agents (e.g., dobutamine, milrinone, amrinone), parenteral loop diuretics, and oxygen. Several landmark studies in the 1980s and early 1990s (e.g., CONSENSUS; SOLVD) showed that ACE inhibitors could lengthen survival and reduce the number of hospitalizations of patients with symptomatic CHF (*N. Eng. J. Med,* 1987;316:1429-1435; and 1991;325:293-302). Even patients with asymptomatic left ventricular (LV) systolic dysfunction were found to benefit by treatment with an ACE inhibitor (SOLVD prevention study). The postulated mechanism is that ACE inhibitors prevent or reduce the upregulation of the renin-angiotensin system (RAS). Unfortunately, no currently available ACE inhibitor is completely effective in halting the progression of heart failure. The majority of CHF patients given optimal treatment with an ACE inhibitor still progress to intractable pump failure or suffer sudden death. As a result, therapies have been directed at other factors associated with progression of heart failure. Increased sympathetic tone and plasma catecholamines are believed to play a role. The degree of functional impairment is roughly correlated with magnitude of sympathetic upregulation. Several β-blockers have been investigated albeit with mixed results. Carvedilol, a nonselective β-blocker, has been shown to lessen combined CHF morbidity and mortality in chronic mild to moderate heart failure. However some patients decompensate during initiation of drug therapy, and its use is not approved in patients with acute heart failure. Furthermore, patients treated with carvedilol plus an ACE inhibitor continue to progress inexorably toward death. Patients with advanced heart failure have limited medical options even though ACE inhibitors and carvedilol are useful adjuncts.

Heart failure may be precipitated acutely by the loss of viable myocardium, but its gradual progression over many years involves many interdependent factors: catecholamines and other hormonal factors (e.g., angiotensin II [Ang II]; aldosterone; arginine vasopressin [AVP]; Endothelin-1 [ET-1]; Atrial Natriuretic Factor [ANF]) are thought to contribute to the pathophysiology of LV enlargement and myocardial "remodeling" (Pauleur, *Am. J. Cardiol.,1994;73:36C-39C).* The benefits of ACE inhibition have pointed to the key role of the renin-angiotensin system (especially Ang II) in LV dilation and heart failure development. However, the progression of heart failure may not involve the same underlying mechanism throughout its course. One set of factors may play a primary role in the onset and early progression of ventricular dysfunction, other substances in the middle phase of symptoms and events, and different factors in the terminal phases of the disease. Furthermore, the benefits and risks of therapeutic interventions may vary with the severity of heart failure. Patients with severe heart failure are most prone to hospitalization and most restricted in their functional capacity. These are the patients that become unresponsive to ACE inhibitors. Of note, serum sodium concentration is an independent prognostic factor for outcome of patients with severe CHF. Hyponatremic patients have a much higher mortality and frequently have serial admissions for heart failure decompensation. These observations suggest that AVP, the neurohormone responsible for regulation of serum osmolality, may be a key factor in progression of heart failure in severely compromised patients.

AVP, a neuropeptide hormone, is synthesized in the hypothalamus, stored in the posterior pituitary, and released into the circulation after activation of neurosecretory cells. There are 2 AVP receptor subtypes. The V_{1A}-subtype mediates contraction in blood vessels and platelet aggregation. V₁ receptors are also involved in the stimulating effect of AVP on adrenocorticotropic hormone (ACTH) secretion. The V₂ receptor is coupled to aquaporine channels in the human kidney and modulates water clearance.

I have now discovered that compounds which inhibit ACE can be used in conjunction with compounds which inhibit vasopressin enzymes to achieve surprisingly good results in treating CHF and related cardiovascular diseases like ventricular dilation, cardiac inefficiency, and hypertension.

### SUMMARY OF THE INVENTION

This invention provides a composition comprised of an ACE inhibitor and a vasopressin antagonist of Formula I or a vasopressin antagonist as defined in claim 8. Any ACE inhibitor can be employed in this invention. In a preferred embodiment, the ACE inhibitor is selected from captopril, enalapril, enalaprilat, lisinopril, ramipril, zofenopril, ceroanapril, alacepril, benazepril, delapril, pentopril, quinapril, quinaprilat, moexipril, rentiapril, quinapril, spirapril, cilazapril, perindopril, and fosinopril.

Vasopressin antagonists are chemical compounds effective in inhibiting the biological activity of any arginine vasopressin or antidiuretic hormone. Numerous compounds are known to be vasopressin antagonists, for example, a condensed benzazepine such as those described in U.S. Patent No. 5,723,606, and specific vasopressin antagonists such as YM471, OPC-31260, OPC-21268, OPC-41061, SR-121463, SR-49059, VPA-985, CL-385004, FR-161282, JVT-605, VP-339, WAY-140288.

The vasopressin antagonist to be used in the present invention is an imidazo benzazepine of the Formula I 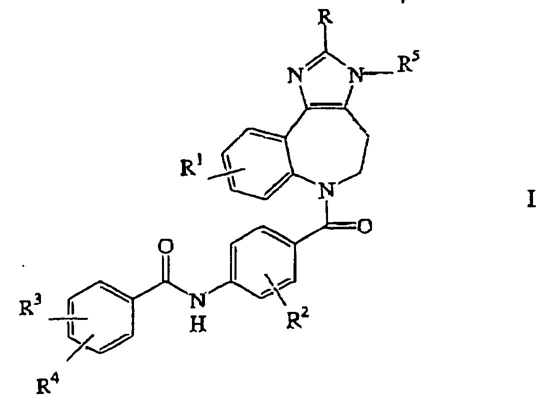 wherein R and R⁵ arc hydrogen or lower alkyl;
R¹, R², and R³ independently are hydrogen, halo, lower alkyl, lower alkoxy, amino, alkylamino, or dialkylamino; and
R⁴ is hydrogen, phenyl or substituted phenyl, and pharmaceutically acceptable salts thereof.

An especially preferred vasopressin antagonist to be used in accordance with this invention is Conivaptan, which is N-[4-(2-methyl-,4,5,6-tetrahydromidazo[4,5-*d*][1]benzazepin-6-ylcarbonyl)phenyl]biphenyl-2-carboxamide hydrochloride. Conivaptan is also referred to as CI-1025, as well as YM087, and has the structural formula below

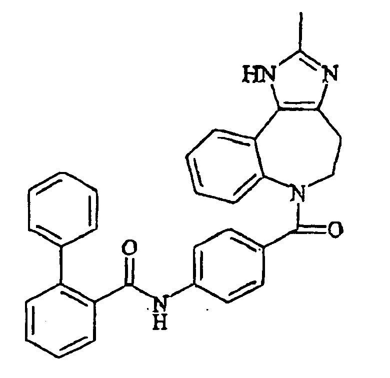

Other vasopressin antagonist to be used in the present invention are compounds selected from the group consisting of:
5-Dimethylamino-1-[4-(2-methylbenzoylamino)-benzoyl]-2,3,4,5-tetrahydro-1H-benzazepine;
5-Dimethylamino-1-[2-chloro-4-(2-methylbenzolamino)benzoyl]-2,3,4,5-tetrahydro-1H-benzazepine;
5-Methylamino-1-[2-chloro-4-(2-methylbenzoylamino)benzoyl]-2,3,4,5-tetrahydro-1H-benzazepine;
5-Cyclopropylamino-1-[2-chloro-4-(7-methylbenzoylamino)benzoxyl]-2,3,4,5-tetrahydro-1H-benzazepine;
5-Cyclopropylamino-1-[2-chloro-4-(2-chlorobenzoylamino)benzoxyl]-2,3,4,5-tetrahydro-1H-benzazepine;
5-Dimethylamino-1-[2-methyl-4-(2-methylbenzoylamino)benzoyl]-2,3,4,5-tetrahydro-1H-benzazepine;
5-Dimelhylamino-1-[2-methoxy-4-(2-methylbenzoylamino)benzoyl]-1,2,3,4-tetrahydroquinoline;
7-Chloro-5-methylamino-1-[4-(2-methylbenzoylamino)benzoxy]-2,3,4,5-tetrahydro-1H-benzazepine; and
7-Chloro-5-methylamino-1-[4-(2-chlorobenzoylamino)benzoxyl]-2,3,4,5-tetrahydro-1H-benzazepine.

The invention also provides a use of an effective amount of the combination of an ACE inhibitor and a vasopressin antagonist of Formula I or a vasopressin antagonist selected from the group mentioned above for the manufacture of pharmaceuticals for treating CHF, ventricular dilation, and hypertension by administering to a mammal in need of treatment.

### BRIEF DESCRIPTION OF FIGURES

Figure 1 shows the reduction in baseline pulmonary capillary wedge pressure (PCWP) caused by various doses of conivaptan in patients receiving an ACE inhibitor.
Figure 2 shows the reduction in right atrial pressure (RAP) caused by various doses of conivaptan in patients receiving an ACE inhibitor.

### DETAILED DESCRIPTION OF THE INVENTION

The ACE inhibitors to be employed in the compositions of this invention are well-known in the art, and several arc used routinely for treating hypertension. For example, captopril and its analogs are described in U.S. Patent Nos. 5,238,924 and 4,258,027. Enalapril, enalaprilat, and closely related analogs are described in U.S. Patent Nos. 4,374,829; 4,472,380; and 4,264,611. Moexipril, quinapril, quinaprilat, and related analogs are described in U.S. Patent Nos. 4,743,450 and 4,344,949. Ramipril and its analogs are described in U.S. Patent Nos. 4,587,258 and 5,061,722. All are typical ACE inhibitors which can be utilized in combination with a vasopressin antagonist according to this invention. Other ACE inhibitors which can be utilized include fosinopril, fasidotril, glycopril, idrapril, imidapril, mixanpril, perindopril, spirapril, spiraprilat, temocapril, trandolapril, zofenopril, zofenoprilat, utilapril, sampatrilat, SA 7060, DU 1777, BMS 186716, and C 112.

The compositions of this invention will contain an ACE inhibitor and a vasopressin antagonist in a weight ratio of about 0.05:1 to about 1000:1, and typically about 1:1 to about 500:1, and ideally about 1:1 to about 5:1. A typical composition, for example, will have 20 mg of quinapril hydrochloride and about 10 mg of conivaptan. All that is required is that amounts of each component are used which are effective to inhibit or reverse CHF, ventricular dilation, or hypertension. The compounds can be administered separately to a patient to effect treatment according to this invention.

As used herein, "patient" means a mammal suffering from a cardiovascular disorder such as CHF and in need of treatment. Patients include humans and animals such as dogs, cats, and sheep. The use of this invention is practiced by administering an effective amount of an ACE inhibitor and a vasopressin antagonist to a patient

An "effective amount" as used herein is the individual quantities of ACE inhibitor and vasopressin antagonist that are routinely used in clinical treatment of hypertension and other cardiovascular disorders. Typical effective amounts will be about 5 to about 500 mg/kg of ACE inhibitor, and about 1 to about 100 mg/kg of vasopressin antagonist. The "effective amount" is that quantity that gives a positive effect in treating the CHF, for example, by causing a reduction in PCWP or in RAP. The precise dosage that is effective according to this invention will be determined by the attending medical practitioner, taking into account the specific ACE inhibitor and vasopressin antagonist being administered, the particular condition of the patient being treated, the duration of the treatment and severity of the disease, and such other factors routinely considered when practicing sound medical judgment.

The combination of an ACE inhibitor with a vasopressin antagonist is synergistic in its ability to treat cardiovascular pathologies such as CHF, as well as hypertension, and left ventricular systolic dysfunction.

Terms used in this specification have the following meanings:
- CHF: Congestive Heart Failure
- NYHA: New York Heart Association
- CI: Cardiac Index
- PCWP: Pulmonary Capillary Wedge Pressure
- SBP: Systolic Blood Pressure
- PVR: Pulmonary Vascular Resistance
- SVR: Systemic Vascular Resistance
- RA: Right Atrial Pressure
- PAs: Pulmonary Artery Systolic Pressure
- PAd: Pulmonary Artery Diastolic Pressure
- DBP: Diastolic Blood Pressure
- MAP: Mean Arterial Pressure
- LV: Left Ventricular
- CAD: Coronary Artery Disease
- ALT: Alanine Aminotransferase
- AST: Aspartate Aminotransferase
- Alk Phos: Alkaline Phosphatase
- LV-EF: Left Ventricular Ejection Fraction
- MuGA: Multi-Gated Radionuclide Ventriculogram
- ACE: Angiotensin Converting Enzyme
- Cr: Serum Creatinine
- BUN: Blood Urca Nitrogen
- WBC: White Blood Cells
- Hgb: Hemoglobin
- ULN: Upper Limit of Normal
- HR: Heart Rate
- CRF: Case Report Form
- COPD: Chronic Obstructive Pulmonary Disease
- FVC: Forced Vital Capacity
- FEV₁: Forced Expiratory Volume in 1 Second

### EXAMPLE 1

The following studies establish the clinical efficacy of YM08 and combinations of ACE inhibitors and vasopressin antagonists.

Preclinical pharmacologic studies have demonstrated potent binding of YM087 conivaptin to AVP receptors and antagonism of the vascular and renal effects of AVP. YM087 has high affinity for V_{1A}- and V₂-receptors with pKi (negative log of the binding inhibition constant) of 8.20 for human V_{1A}-receptors and 8.95 for human V₂-receptors expressed in COS-1 cells.

### Clinical Pharmacology

YM087 given orally to rats antagonizes the AVP-induced pressor response (V_{1A} antagonism) in a dose-related manner, with the dose that reduced the AVP response by 50% (ID₅₀) being 0.32 mg/kg; ID₅₀ for a similar experiment using intravenous (IV) YM087 in dogs was 0.026 mg/kg. In conscious dogs, oral YM087 (0.03 to 0.3 mg/kg) increased urinary output (V₂ antagonism) and reduced urinary osmolality (from 1500 to <100 mOsm/kg H₂O) in a dose-related manner. Unlike furosemide, YM087 has little or no effect on urinary sodium (Na) or potassium (K) excretion. In dogs with heart failure induced by rapid right ventricular pacing, intravenous administration of YM087 (0.1 mg/kg) significantly improved the depressed cardiac function and produced a water diuresis.

Oral absorption of YM087 is rapid (peak concentrations reached between 0.5 to I hour in the rat and dog, respectively) and occurs predominantly in the small intestine. There is a marked food effect with absorption reduced by >50% in dogs after a meal. The elimination half-life is 1 hour in rats and 2 hours in dogs. Mass balance studies show the majority of radioactive tracer excreted in the feces.

The preclinical toxicologic potential af YM087 has been extensively evaluated, and all findings were evaluated for relevance to human risk assessment and impact on clinical trial design. Findings of potential concern were bone marrow changes in dogs and effects on fertility in rats.

Histopathologic changes in bone marrow were observed in both 2- and 13-week oral studies in dogs with systemic exposures 28- to 87-fold higher than the maximum anticipated human exposure. Decreased peripheral erythrocyte, leukocyte, and/or platelet counts occurred in affected dogs in the 13-week study. Bone marrow and peripheral blood changes were reversible.

YM087 did not affect reproductive performance of male rats. In the 13-week, repeated oral dose study in rats, more females at 10 mg/kg were in diestrus or proestrus and fewer were in estrus than in controls, and uterine weights were decreased at all doses; associated systemic exposures were 0.06- to 3.2-fold the maximum anticipated human exposure. In the female fertility study in rats, reduced fertility index, increased implantation loss, and decreased live fetuses were observed in females given 100 mg/kg orally for 2 weeks prior to mating with untreated males. Effects on estrous cycle and fertility in female rats may be related to alterations in serum hormone levels resulting from pharmacologic activity of YM087. YM087 was not teratogenic in rats or rabbits.

Other drug-related effects, including diuresis and hepatocellular hypertrophy, were of less concern due to the nature of the effects or the high exposures at which the effects occurred compared to exposures anticipated in clinical trials.

YM087 was not mutagenic in bacteria, and was not clastogenic in human lymphocytes in vitro or in bone marrow of rats. No toxicity was observed in 4-week, IV studies with the glycerin formulation at maximum achievable doses, 2.5 mg/kg in rats and 2 mg/kg in dogs.

In summary, toxicological findings of potential concern for human risk assessment were reversible effects on bone marrow in dogs and reversible effects on estrus cycle and decreased fertility in rats. Findings in bone marrow were observed at exposures in excess of 23 times exposure expected in humans given the maximum dose of 120 mg once daily (QD), while effects on estrus cycle occurred at exposures from 0.05- to 3-fold the expected human exposure at 120 mg QD. Other drug-related findings in toxicology studies were considered secondary to pharmacologic activity or a functional adaptation to exposure to YM087.

YM087 has been given to approximately 250 healthy subjects who participated in a total of 15 Phase 1 studies (8 in Japan and 7 in Europe). Subjects taking oral medication received either a single dose of YM087 (dose range 02 through 120 mg) QD or 30 or 120 mg YM087 administered as a divided dose twice daily (BID). Subjects received YM087 as a single IV injection once daily over a dose range of 0.2 to 250 µg/kg or up to a maximum of 50 mg.

Inhibition of AVP-induced platelet aggregation (evidence of V_{1A} antagonist activity) was seen among subjects who received YM0137 at 20 mg/day orally or 2.5 mg IV. Total inhibition of AVP-induced dermal vasoconstriction was observed among subjects who received YM087 50 mg IV

Normal subjects have demonstrated aquaretic action (evidence of V₂-receptor antagonism) accompanied by a decrease in urine osmolarity starting at 15 mg oral or 50 µg/kg IV. At higher doses aquaretic effects were more pronounced and at 120 mg QD or 60 mg BID given orally or 50 mg given IV were considered too uncomfortable in normal subjects to be tolerable. YM087 at IV doses up to 250 µg/kg and 50 mg/day increased urine production rate for up to 3 and 6 hours postdosing, respectively.

Under fasting conditions, YM087 is rapidly absorbed, time to maximum plasma concentration (tmax) being reached at around 1 hour. The mean oral bioavailability of a 60-mg dose is 44% under fasting conditions; bioavailability is decreased after intake with food. A high-fat breakfast reduced bioavailability of single 15- to 90-mg doses of YM087 to 43% to 59% of the fasted value, and peak plasma levels were reduced to 24% to 54% of the fasting value. Oral YM087 demonstrated a nonlinear pharmacokinetic profile. Repeated BID oral doses of YM087, 60 mg, result in unexpectedly high plasma levels after the second dose, possibly caused by reduced first-pass metabolism. YM087 displays 2 compartment pharmacokinetics, with an elimination half-life of 4 to 5 hours. Elderly subjects have a similar elimination half-life as healthy, young volunteers.

The pharmacokinetics of orally administered YM087 (20 mg) were not affected when combined with either 0.5 mg IV digoxin or 25 mg oral captopril (each given as a single dose).

### Safety

Among approximately 250 subjects treated, no major safety concerns were identified. One patient with severe CHF who received YM087 80 mg/day for 4 days experienced a generalized tonic clonic seizure, which the investigator could not exclude as related to study drug. The most frequent adverse events regardless of treatment association were mild or moderate thirst and mild headache. Other adverse events included flushes, a sensation of cold extremities, abdominal complaints, abnormal stools, syncope, dizziness, palpitations, and postural hypotension. Three subjects who received YM087 and one subject who received placebo developed minor, reversible leukopenia. No drug-related trend was observed in biochemical or hematological laboratory parameters. At higher doses, urinary osmolarity decreased and plasma osmolarity increased with or without an increase in plasma sodium. These observations were considered related to antagonism of V₂ receptors and not a safety concern. Vital signs (blood pressure and heart rate) were unaffected by YM087.

### EXAMPLE 2

### ACE + Vasopressin Antagonists, in CHF

This trial is a double-blind, placebo-controlled study of the intravenous dose response of YM087 on cardiopulmonary hemodynamics in 142 patients with Class III/IV heart failure. These patients have advanced CHF/LV dysfunction. Patients must be receiving background therapy of diuretics, ACE inhibitors, and optionally digoxin and/or β-blocker; patients will be stratified as to whether they are receiving concomitant β-blocker treatment. Eighty-five percent of the patients in this study received an ACE inhibitor and YM087. Patients should take their daily dose of concomitant heart failure medications within 2 hours of catheter insertion. No additional doses of background heart failure medications should be administered during the study treatment phase. After insertion of a balloon-floatation pulmonary artery catheter, serial measurements will be obtained over an 8- to 18-hour baseline and stabilization period Patients meeting baseline eligibility criteria (CI ≤2.6 L/min/m²; PCWP ≥16 mmHg) after catheter stability is assured will be administered an IV dose (30-minute infusion) of YM087 or placebo and monitored for the subsequent 12 hours. The mean baseline PCWP in this study group was 24.2 mmHg. The mean baseline CI was 2.1 L/min/m². Hemodynamic parameters and vital signs will be measured at baseline during the 2 hours prior to drug administration and 30 minutes, 1,2,3,4,6,8, and 12 hours after start of the IV infusion. A urethral cathether will be placed and urine output will be measured hourly for 2 hours prior until 12 hours poststudy drug administration. Fluid intake will be restricted to 250 mL every 2 hours (except at time of IV infusion) from time of insertion of Swan-Cranz catheter and throughout the treatment period. YM087 plasma levels will be determined at 1,3, and 8 hours posttreatment. Serum electrolytes, BUN, creatinine, and serum osmolality will be measured at baseline and 4 and 12 hours posttreatment Clinical laboratory and vasopressin plasma levels will be measured at baseline and 12 hours postdrug administration. A numeric rating scale for assessing dyspnea will be administered at baseline and 12 hours after study drug administration. The dosing and analysis schedule used in this study is shown in Table 1.

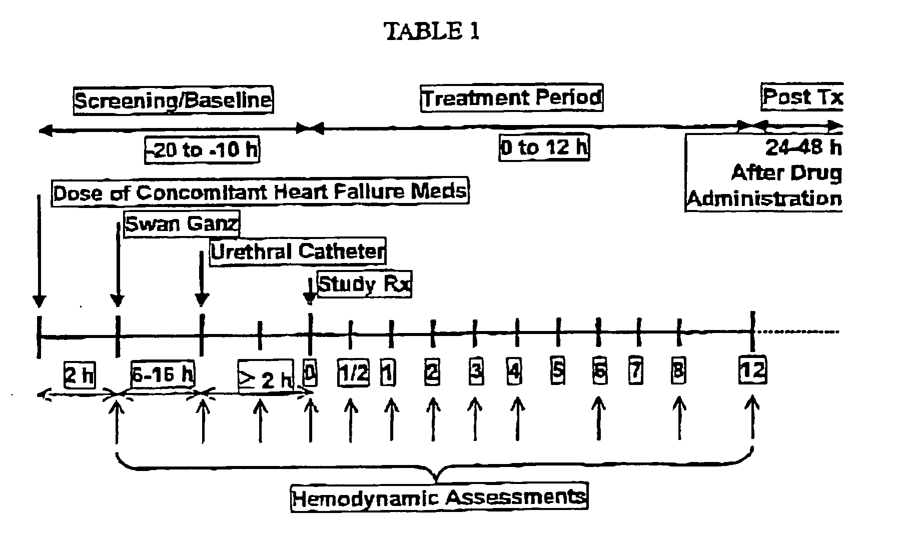

### Screening and Baseline Phase (10-20 Hours)

This phase allows the investigator to evaluate patients who may qualify for entry into the treatment period and to assess baseline values for a number of study parameters. An informed consent must be signed. A medical history, physical examination, and assessment of NYHA functional class will be done. Clinical laboratory parameters will be measured. If left ventricular ejection fraction (LV-EF) has not been measured during the previous 3 months, the patient will undergo radionuclide, contrast ventriculography, or 2-dimensional echocardiography to measure LV-EF.

Patients must remain on stable doses of background heart failure medications throughout the baseline and treatment phase. Patients should take a dose of their concomitant heart failure medications within 2 hours of Swan-Ganz catheter insertion. No additional dose of background medications should be administered during the study treatment phase. After insertion of a balloon-floatation pulmonary artery catheter, several measurements of hemodynamic parameters will be obtained over a 8- to 18-hour baseline and stabilization period. Patients meeting baseline eligibility criteria (CI ≤2.6 L/min/m²; PCWP ≥16 mmHg) on successive readings at least 30 minutes apart during 2 hours prior to study drug administration will enter the treatment phase. Additional measurements of hemodynamic parameters over a larger baseline period (≥2 hours) may be required to meet the reproducibility criteria. The 2 successive measurements of PCWP and CO must be ±10% and ±15%, respectively, of the mean. Patients should fast 6 hours prior to baseline measurements and must remain fasting for the first 6 hours of the 12-hour treatment phase.

Patients who qualify for entry will have blood drawn for a baseline assessment of clinical laboratory and vasopressin plasma levels. A urethral cathether should be placed and urinary output measurements will be obtained hourly for ≥2 hours prior to study drug administration and thereafter during the treatment phase. Hemodynamic measurements should be obtained at least 30 minutes after insertion of the urethral catheter. Fluid intake will be restricted to 250 mL every 2 hours (except at time of IV infusion) from time of insertion of Swan-Ganz catheter and throughout treatment period. Vital signs will be assessed at least every 4 hours. A numeric rating scale for assessing dyspnea will be administered within 1 hour of study drug administration.

### Treatment Phase (12 Hours):

Patients meeting baseline eligibility criteria will be randomized within an hour to receive double-blind IV bolus dose, administered over 30 minutes, of placebo or 1 of 3 doses of YM087 (10, 20, or 40 mg) in a 1:1:1:1 ratio. Patients should refrain from taking concomitant heart failure medications during the 12-hour treatment period. Patients will be stratified as to whether they are receiving concomitant treatment with a β-blocker. Hemodynamic parameters (cardiac output, intrapulmonary and systemic pressures) and vital signs will be measured at 0.5, 1, 2, 3, 4, 6, 8, and 12 hours after start of the IV infusion. Clinical laboratory and vasopressin plasma levels will be measured 12 hours postdose. YM087 plasma levels will be determined at 1, 3, and 8 hours posttreatment. Serum electrolytes, BUN, creatinine, serum osmolality will be measured at 4 and 12 hours posttreatment. Hourly urine output measurements will be obtained during the entire 12-hour treatment phase. The numeric rating scale for assessing dyspnea will be administered 12 hours postadministration of study medication.

### Posttreatment Phase (24-48 Hours) After Administration of Study Medication:

Patients must return for an outpatient follow-up visit at least 24 to 48 hours after administration of study medication. Patients will be followed for clinical assessment of adverse events. Clinical laboratory parameters will be measured. Background heart failure medications will be readjusted if necessary for safety/tolerance of the patient

### STUDY POPULATION

All patients enrolled into this study will have NXHA, Class III/IV heart failure due to systolic LV dysfunction.

### Source and Number of Patients

A total of 142 patients (35 per treatment group) will be enrolled at 20 study canters. Each site is expected to enroll 6 to 8 patients. Enrollment is competitive and will stop when the study is complete.

### Patient-Selection Criteria

### Inclusion Criteria

Patients acceptable for inclusion into the study must meet the following criteria:
- Males or females 18 to 80 years of age; females must be postmenopausal, surgically sterilized, or practicing a suitable method of birth control so that in the opinion of the investigator, they will not become pregnant during the study;
- Symptomatic heart failure with Class III/IV functional impairment by NYHA criteria;
- Current therapy for heart failure consisting of at least 1 month duration of an ACE inhibitor, loop diuretic, and optionally digoxin and/or β-blocker,
- Cardiac index ≤2.6 L/min/m²; and PCWP ≥16 mmHg on successive readings at least 30 minutes apart prior to study drug administration; and
- Signed informed consent.

### Exclusion Criteria

Presence of any of the following conditions will exclude the patient from being eligible for study:
- Breast feeding or pregnant;
- Patients with supine systolic blood pressure <95 mmHg or uncontrolled hypertension;
- Patients with more than 2+ edema (above the knee);
- Uncontrolled symptomatic brady- or tachyarrhythmias (e.g., sinus arrest second-degree Mobitz type II) or third-degree AV block; atrial fibrillation or flutter; frequent runs of ventricular tachycardia); patients with dual chamber pacemakers and/or implantable defibrillators are eligible, if the device has been implanted >60 days prior to screening;
- Unstable angina pectoris and/or acute myocardial infarction within 1 month of baseline;
- Patients with severe COPD (FVC ≤1.5 L; FEV₁ ≤1.0 L) or primary pulmonary hypertension;
- Patients with significant uncorrected primary valvular disease or uncorrected congenital heart disease; for example, aortic stenosis (AVA <0.8 cm²), mitral stenosis (MVA <1.2 cm²/m²), severe valvular insufficiency requiring valve replacement;
- Patients with obstructive cardiomyopathy;
- Patients with active myocarditis, constrictive pericarditis, untreated hypothyroidism or hyperthyroidism, adrenal insufficiency, active vasculitis due to collagen vascular disease, or other correctable nutritional or metabolic causes for heart failure;
- Alanine aminotransferase (ALT) and aspartate aminotransferase (AST) elevations >3 times the upper limit of normal (ULN) reference range and/or bilirubin ≥2 mg/dL;
- Patients with significant renal impairment; serum creatinine >2.5 mg/dL or creatinine clearance <30 mL/min;
- Serious hematological diseases (e.g., severe anemia, Hgb <10 g/dL:leukopenia, white blood cell [WBC] <4000/µL);
- Active cancer within 5 years of screening for this study (exclusive of localized skin cancer or localized prostate cancer);
- Patients on continuous and/or daily doses of IV inotropic drugs (e.g., dobutamine; dopamine, milrinone, amrinone, etc) or parenteral vasodilators (e.g., nitroprusside; nitroglycerin) within 7 days of screening;
- Clinical evidence of digitalis toxicity;
- Current illicit drug use or alcoholism;
- Any concurrent illness which, in the opinion of the investigator, may interfere with treatment, evaluation of safety, and/or efficacy,
- Participation in another clinical trial of an investigational drug (including placebo) within 30 days of screening for entry into the present study, or
- Inability to understand and sign the Informed Consent to participate in this study.

### Prohibited/Allowable Medications or Precautions

To minimize confounding factors and bias in interpreting results related to potential cardiac changes not associated with natural progression of CHF, concurrent heart failure medications should be held stable throughout the treatment phase of the study. Changes in concurrent medications can and should be made where issues of patient safety are evident.

Nonsteroidal anti-inflammatory agents (NSAIDS) are discouraged duc to their inhibitory effects on renal function.

Permitted medications include those used to treat coronary artery disease (CAD), hypertension, diabetes, hyperlipidemia, and CHF. Heart failure medications can include ACE inhibitors, diuretics, digoxin, β-blocker, and intermittent oxygen. No other parenteral vasodilators (e.g., nitroprusside, nitroglycerin) nor initiation of inotropic agents will be allowed. Chronic low dose (≤300 mg QD) amiodarone is permissible but not sotalol, dofetilide or other Class III antiarrhythmic agents. Calcium channel blockers with negative inotropic effects (e.g., verapamil, diltiazem) are prohibited.

Patients enrolled in this study cannot be participating in any other ongoing protocol studying the effects of investigational medications.

### Meals and Fluid Intake

Patients should fast at least 6 hours prior to baseline hemodynamic measurements and must remain fasting during the first 6. hours of the 12-hour treatment phase.

Fluid intake will be restricted to 250 mL every 2 hours (except at time of IV infusion of study medication) from time of insertion of Swan-Ganz catheter and throughout the treatment period.

### STUDY METHODOLOGY

### Efficacy Parameters

### Primary Efficacy Parameter

Peak change from last baseline measurement in PCWP at 3 to 6 hours after start of study medication infusion as compared to placebo. Other characteristics of response profile (area under the PCWP/time curve) are defined in statistical analysis section of the protocol.

### Secondary Efficacy Parameters

- Peak change from last baseline measurement at 3 to 6 hours after start of study medication infusion as compared to placebo in:
   - Cardiac index (CI)
   - Pulmonary vascular resistance (PVR)
   - Systemic vascular resistance (SVR)
- Other characteristics of response profile (CI, PVR, SVR) are defined in the statistical analysis section of the protocol (area under the curve [AUC]).
- Changes in urine output over time as compared to baseline.
- Descriptive statistics for RA, PAs, PAd, BP, HR will also be performed.
- Change from baseline in numeric rating scale for assessing dyspnea at 12 hours after administration of study medication.

### Safety Assessments

- Hemodynamic Parameters: Boundary values for reduced cardiovascular performance are (expressed as changes from baseline demonstrated on 2 successive readings 230 minutes apart): (a) >25% decrease in CI from baseline; (b) ≥6 mmHg rise in PCWP above baseline; and (c) systolic arterial BP (SBP) <80 mmHg or >10 mmHg fall in SBP associated with presyncopal symptoms;
- Changes in serum electrolytes;
- Clinical laboratory measures including renal function (BUN and creatinine), liver function tests (ALT, AST, Alk Phos, bilirubin), hematological parameters (WBC, neutrophils);
- Adverse events;
- Changes in urine output over time.

### Pharmacokinetic/Pharmacodynamic Analysis

Plasma concentrations of YM087 will be measured at 1, 3, and 8 hours after start of IV infusion of study medication using a validated LC/MS/MS method. Assay sensitivity, specificity, linearity, and reproducibility will be determined before analysis of samples.

The relationship between the primary efficacy parameters and plasma concentrations of YM087 including the interindividual variability will be evaluated using appropriate pharmacostatistical methods.

### Neurohormonal Assessments

Vasopressin plasma levels will be measured at baseline and 12 hours after start of IV infusion of study medication using standard methods.

### Dosing Procedure

YM087 sterile injection will be added to a 50-mL bag containing D5W. Table 2 specifies the amount of YM087 for injection to dilute into D5W in order to achieve the desired dose. The contents of the bag will be administered to the patients via a pump infusion system (e.g., IMED^{™}, IVAC^{™}) over 30 minutes.

**TABLE 2. YM087 Dose Administration**

| Dose (mg) | Volume of YM087 (mL) | Volume of D5W added (mL) | Total Volume in Bag (mL) | Concentration (mg/mL) | Infusion Rate Over 30 Minutes (mL/min) |
|---|---|---|---|---|---|
| 10 | 2 | 8 | 60 | 0.167 | 2 |
| 20 | 4 | 6 | 60 | 0.333 | 2 |
| 40 | 8 | 2 | 60 | 0.667 | 2 |

### STATISTICAL ANALYSIS AND RATIONALE

### Power and Sample Size

For this study, a total of 140 patients will be considered with 35 patients in each of the 4 treatment groups (placebo, 10, 20, and 40 mg). The power to detect a difference of 3 mmHg in the PCWP peak change from baseline within 3 to 6 hours after treatment administration, between placebo and any of the 3 active treatments, was determined using the formula for the power of the t-test. Adjustment for multiple comparisons with placebo was performed using Dunnett's approach (Dunnett, *Biometrics,* 1964;20:482-491). Assuming a 15% dropout rate, an overall error rate of 0.05 two-sided, and a standard deviation of 3 mmHg for the PCWP peak change, the power to detect a difference of 3 mmHg between placebo and any of the YM087 groups is 93.6%. However, if a standard deviation of 4 mmHg is assumed, the power becomes 71.1%.

### Efficacy Parameters

Hemodynamic data in clinical trials of congestive heart failure is usually assessed by evaluating the differences in change from baseline to peak response among treatment groups. Generally, peak response is defined as an average of measurements taken at prespecified hours (e.g., at 2,3, and 4 hours).

For this study, hemodynamic efficacy parameters of PCWP, Cl, SVR, and PVR will be evaluated in terms of their response profile. The response profile will be assessed in terms of peak change, and AUC delimited by the parameter change from baseline and measurement times. The peak change is defined as the maximum change from baseline, within the 3 to 6 hours after treatment administration, in the hemodynamic parameter of interest. The baseline value is considered the last acceptable measurement taken before treatment administration. The AUC will be determined using the "linear trapezoidal rule", by which areas of each trapezoid delimited by: 2 points on the graph of change from baseline against time, perpendiculars from the points to the X-axis, and the X-axis are summed up to get AUC. If measurements are missing at certain times, the AUC will be calculated using all other available observations. Dose response over the treatment groups will be assessed for selected measures.

The primary efficacy parameter for this study is peak change from baseline in PCWP. The secondary parameters are peak change from baseline in CI, SVR, and PVR. Changes in RA and PA pressures also will be characterized. In addition, changes in urine output will be characterized.

### Analysis of the Primary Efficacy Parameter

An analysis of covariance (ANCOVA) model will be used as the primary analysis to compare each of the YM087 doses with placebo in terms of peak change in PCWP. The model will include effects due to treatment, center, an indicator variable for the presence or absence of β-blocker therapy, and possibly the baseline value as a covariate. Treatment-by-center and treatmeat-by-baseline interactions will be investigated. All randomized patients that have a baseline measurement and at least one follow-up measurement will be considered for this analysis. If there is only one observation within 3 to 6 hours then the peak change will be calculated using that observation and the baseline value. If there are no measurements in the 3 to 6 hour window, the last measurement prior to hour 3 will be carried forward and used to calculate peak.

A secondary analysis of the AUC will be conducted to support the primary analysis. For the area under the PCWP change from baseline and time curve, the analysis will be conducted using ANCOVA in a similar manner as described for the primary analysis. The model will include effects due to treatment, center, an indicator variable for the presence or absence of β-blocker therapy, and possibly the baseline value as a covariate. Treatment-by-center and treatment-by-baseline interactions will be investigated. All randomized patients that have a baseline and at least one follow-up measurement will be considered for this analysis.

In order to claim positivity, results from the primary analysis for peak change in PCWP should be significant at the a level corresponding to 0.049 using Dunnett's approach, or results from the secondary analysis of AUC at the 0.001 level. A supportive secondary trend analysis for dose response will also be performed. Also, repeated measures ANCOV will be performed for selected measurements of the response profile.

### Analysis of the Secondary Efficacy Parameters

The primary analysis for the secondary efficacy parameters of CI, SVR, and PVR will be performed using ANCOVA as described for the primary efficacy parameter, to compare the treatment groups with placebo in terms of their peak change from baseline. Patients will be considered for this analysis according to the criteria described for the primary parameter. The significance levels will be adjusted for multiple comparisons with placebo using Dunnett's method.

Analysis of the AUC and trend analysis will be considered supportive, and conducted in the same manner as described for the primary parameter. Repeated measures ANCOVA will be performed for selected measurements of the response profile. All randomized patients with a baseline and at least one follow-up measurement will be considered.

The secondary parameter of urine output will be summarized at baseline, and each collection time. In addition, a numeric rating scale will be used for assessing dyspnea The corresponding change from baseline for these parameters will be summarized. Descriptive summaries will include mean, standard error, median, minimum, and maximum. Other concurrently measured hemodynamic parameters (i.e., RA, PAs, PAd, cuff SBP, cuff DBP, calculated MAP, and HR) will also be summarized.

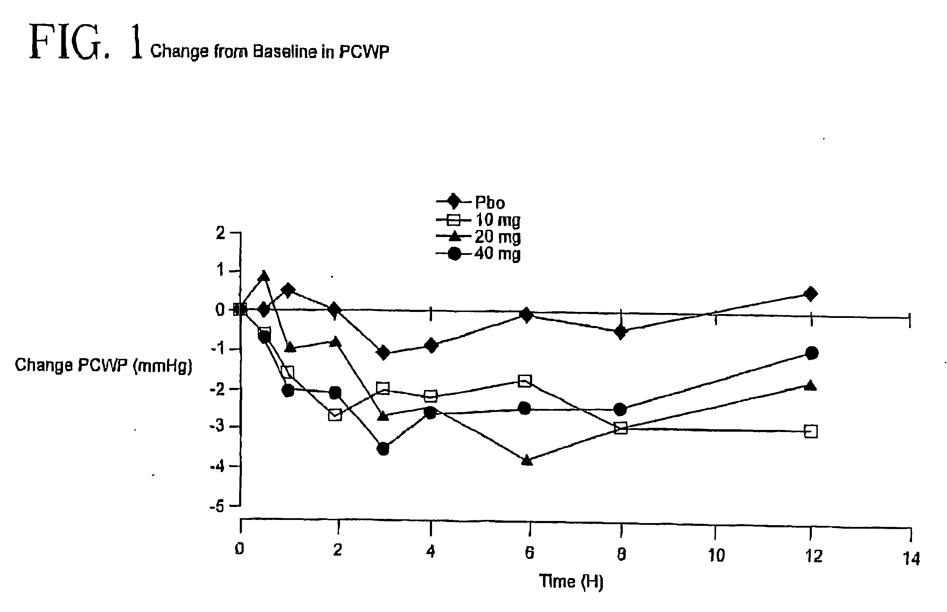

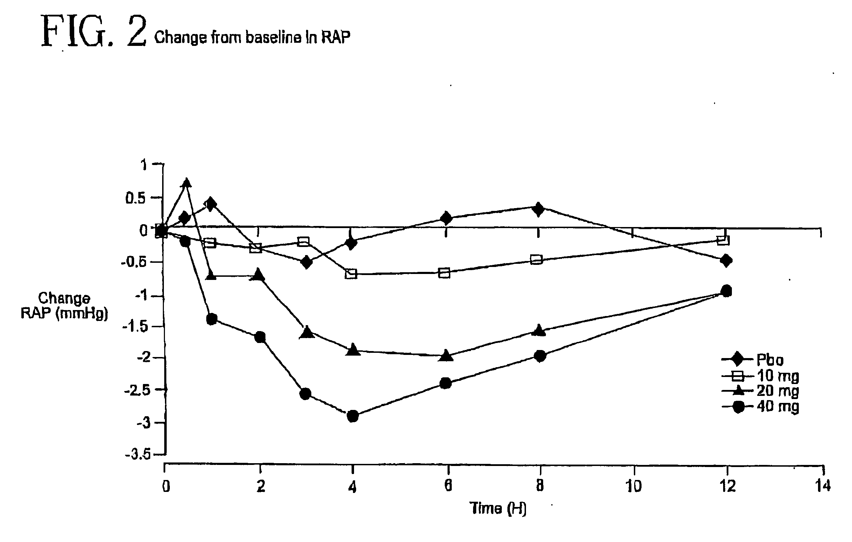

The results of the foregoing study establish that conivaptan has a surprisingly favorable hemodynamic effect as an add-on therapy to normal treatment with ACE inhibitors. Eighty-five percent of the patients in this study were treated with ACE inhibitors (plus conivaptan). As shown in Figure 1, conivaptan caused significant reductions in PCWP. Figure 2 shows that conivaptan caused a significant reduction in RAP.

The compositions to be employed in the present invention can be prepared and administer in a wide variety of oral and parenteral dosage forms for treating and preventing heart failure and ventricular dilation. The compounds can be administered by injection, that is, intra-venously, intramuscularly, intracutaneously, subcutaneously, submucosally, intraductally, intraduodenally, or intraperitoneally. Also, the compounds can be administered by inhalation, for example, intranasally. Additionally, the compositions can be administered transdermally. It will be obvious to those skilled in the art that the following dosage forms may comprise as the active component, either a compound as a free base, acid, or a corresponding pharmaceutically acceptable salt of such compound. The active compound generally is present in a concentration of about 5% to about 95% by weight of the formulation.

For preparing pharmaceutical compositions from the compounds of the present invention, pharmaceutically acceptable carriers can be either solid or liquid, Solid form preparations include powders, tablets, pills, capsules, cachets, suppositories, and dispersible granules. A solid carrier can be one or more substances which may also act as diluents, flavoring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding properties in suitable proportions and compacted in the shape and size desired.

The powders and tablets preferably contain from 5% or 10% to about 70% of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogenous mixture is then poured into convenient sized molds, allowed to cool, and thereby to solidify.

Liquid form preparations include solutions, suspensions, and emulsions, for example, water or water propylene glycol solutions. For parenteral injection, liquid preparations can be formulated in solution in aqueous polyethylene glycol solution.

Aqueous solutions suitable for oral use can be prepared by dissolving the active component in water and adding suitable colorants, flavors, stabilizing, and thickening agents as desired.

Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included arc solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of each active component in a unit-dose preparation may be varied or adjusted from 1 to 1000 mg, preferably 10 to 100 mg according to the particular application and the potency of the active component. The composition can, if desired, also contain other compatible therapeutic agents.

The following examples illustrate typical formulations that can be utilized in the invention.

**Tablet Formulation**

| Ingredient | Amount (mg) |
|---|---|
| Conivaptan | 25 |
| Quinapril hydrochloride | 20 |
| Lactose | 30 |
| Cornstarch (for mix) | 10 |
| Cornstarch (paste) | 10 |
| Magnesium stearate (1%) | 5 |
| Total | 100 |

The conivaptan, ACE inhibitor, lactose, and cornstarch (for mix) are blended to uniformity. The cornstarch (for paste) is suspended in 200 mL of water and heated with stirring to from a paste. The paste is used to granulate the mixed powders. The wet granules are passed through a No. 8 hand screen and dried at 80°C. The dry granules are lubricated with the 1 % magnesium stearate and pressed into a tablet. Such tablets can be administered to a human from one to four times a day for treatment of CHF.

**Preparation for Oral Solution**

| Ingredient | Amount |
|---|---|
| YM-471 | 400 mg |
| Quinapril | 20 mg |
| Sorbitol solution (70% N.F.) | 40 mL |
| Sodium benzoate | 20 mg |
| Saccharin | 5 mg |
| Red dye | 10 mg |
| Cherry flavor | 20 mg |
| Distilled water q.s. | 100 mL |

The sorbitol solution is added to 40 mL of distilled water, and the vasopressin antagonist and ACE inhibitor are dissolved therein. The saccharin, sodium benzoate, flavor, and dye are added and dissolved. The volume is adjusted to 100 mL with distilled water. Each milliliter of syrup contains 4 mg of invention composition.

### Parenteral Solution

In a solution of 700 mL of propylene glycol and 200 mL of water for injection is suspended 20 g of vasopressin antagonist OPC-31260 and 5 g of enalaprilat. After suspension is complete, the pH is adjusted to 6.5 with 1N sodium hydroxide, and the volume is made up to 1000 mL with water for injection. The formulation is sterilized, filled into 5.0 mL ampoules each containing 2.0 mL, and sealed under nitrogen.

## Claims

1. A pharmaceutical composition comprising an effective amount of an angiotensin-converting enzyme inhibitor and an effective amount of a vasopressin antagonist of Formula I 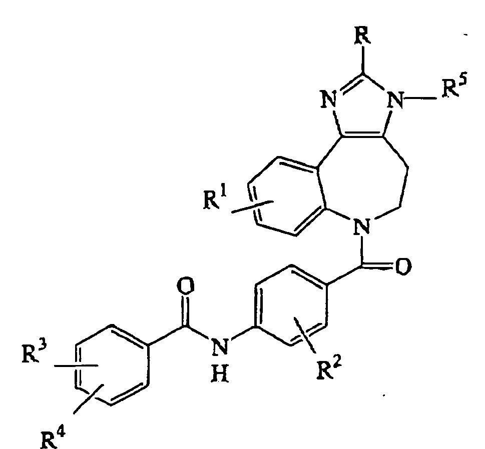 wherein R and R⁵ are hydrogen or lower alkyl;
R¹, R², and R³ independently are hydrogen, halo, lower alkyl, lower alkoxy, amino, alkylamino, or dialkylamino; and
R⁴ is hydrogen, phenyl or substituted phenyl, and pharmaceutically acceptable salts thereof.

2. A composition according to Claim 1 employing an ACE inhibitor selected from captopril, enalapril, enalaprilat, lisinopril, ramipril, zofenopril, trandolapril, temocapril, ceranapril, alacepril, delapril, pentopril, quinapril, quinaprilat, moexipril, rentiapril, duinapril, spirapril, cilazapril, perindopril, and fosinopril.

3. A composition according to Claim 1 in which the vasopressin antagonist is conivaptan.

4. A composition according to Claim 2 comprising an ACE inhibitor selected from quinapril hydrochloride, ramipril, enalapril, or moexipril.

5. A composition according to Claim 4 comprising a vasopressin antagonist selected from conivaptan or a salt thereof.

6. A composition comprising conivaptan and quinapril.

7. Use of a composition according to claims 1 to 6 for the manufacturing of pharmaceuticals for treating ventricular dilation, and/or heart failure.

8. A pharmaceutical composition comprising an effective amount of an angiotensin-converting enzyme inhibitor and an effective amount of a vasopressin antagonist selected from
5-Dimethylamino-1-[4-(2-methylbenzoylamino)-benzoxyl]-2,3,4,5-tetrahydro-1H-benzazepine;
5-Dimethylamino-1-[2-chloro-4-(2-methylbenzoylamino)benzoxyl]-2,3,4,5-tetrahydro-1H-benzazepine;
5-Methylamino-1-[2-chloro-4-(2-methylbenzoylamino)benzoxyl]-2,3,4,5-tetrahydro-1H-benzazepine;
5-Cyclopropylamino-1-[2-chloro-4-(2-methylbenzoylamino)benzoxyl]-2,3,4,5-tetrahydro-1H-benzazepine;
5-Cyclopropylamino-1-[2-chloro-4-(2-chlorobenzoylamino)benzoxyl]-2,3,4,5-tetrahydro-1H-benzazepine;
5-Dimethylamino-1-[2-methyl-4-(2-methylbenzoylamino)benzoxyl]-2,3,4,5-tetrahydro-1H-benzazepine;
5-Dimethylamino-1-[2-methoxy-4-(2-methylbenzoylamino)benzoxyl]-1,2,3,4-tetrahydroquinoline;
7-Chloro-5-methylamino-1-[4-(2-methylbenzoylamino)benzoxyl]-2,3,4,5-tretrahydro-1H-benzazepine; and
7-Chloro-5-methylamino-1-[4-(2-chlorobenzoylamino)benzoxyl]-2,3,4,5-tetrahydro-1H-benzazepine.

9. Use of a composition according to claim 8 for the manufacturing of pharmaceuticals for treating ventricular dilation, and/or heart failure.

## Patentansprüche

1. Pharmazeutische Zusammensetzung, die eine wirksame Menge eines Inhibitors des Angiotensin Converting Enzyme und eine wirksame Menge eines Vasopressinantagonisten der Formel I 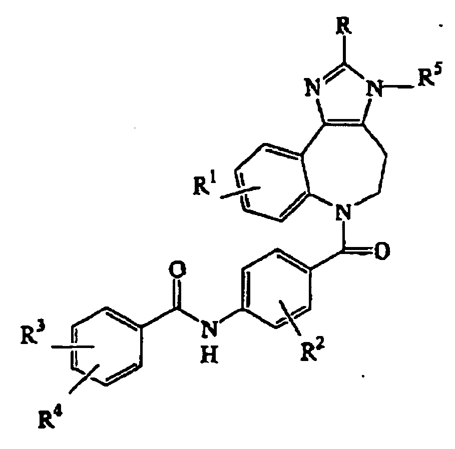
worin R und R⁵ für Wasserstoff oder Niederalkyl stehen;
R¹, R² und R³ unabhängig voneinander für Wasserstoff, Halogen, Niederalkyl, Niederalkoxy, Amino, Alkylamino oder Dialkylamino stehen und
R⁴ für Wasserstoff, Phenyl oder substituiertes Phenyl steht, und pharmazeutisch akzeptabler Salze desselben umfasst.

2. Zusammensetzung nach Anspruch 1 unter Verwendung eines ACE-Inhibitors, der aus Captopril, Enalapril, Enalaprilat, Lisinopril, Ramipril, Zofenopril, Trandolapril, Temocapril, Ceranapril, Alacepril, Delapril, Pentopril, Quinapril, Quinaprilat, Moexipril, Rentiapril, Duinapril, Spirapril, Cilazapril, Perindopril und Fosinopril ausgewählt ist.

3. Zusammensetzung nach Anspruch 1, wobei der Vasopressinantagonist Conivaptan ist.

4. Zusammensetzung nach Anspruch 2, die einen ACE-Inhibitor umfasst, der aus Quinaprilhydrochlorid, Ramipril, Enalapril oder Moexipril ausgewählt ist.

5. Zusammensetzung nach Anspruch 4, die einen Vasopressinantagonisten umfasst, der aus Conivaptan oder einem Salz dessselben ausgewählt ist.

6. Zusammensetzung, die Conivaptan und Quinapril umfasst.

7. Verwendung einer Zusammensetzung nach den Ansprüchen 1 bis 6 zur Herstellung von Arzneimitteln zur Behandlung von Ventrikeldilatation und/oder Herzinsuffizienz.

8. Pharmazeutische Zusammensetzung, die eine wirksame Menge eines Inhibitors des Angiotensin Converting Enzyme und eine wirksame Menge eines Vasopressinantagonisten, der aus
5-Dimethylamino-1-[4-(2-methylbenzoylamino)-benzoyl]-2,3,4,5-tetrahydro-1H-benzazepin,
5-Dimethylamino-1-[2-chlor-4-(2-methylbenzoylamino)-benzoyl]-2,3,4,5-tetrahydro-1H-benzazepin,
5-Methylamino-1-[2-chlor-4-(2-methylbenzoylamino)-benzoyl]-2,3,4,5-tetrahydro-1H-benzazepin,
5-Cyclopropylamino-1-[2-chlor-4-(2-methylbenzoylamino)-benzoxyl]-2,3,4,5-tetrahydro-1H-benzazepin,
5-Cyclopropylamino-1-[2-chlor-4-(2-chlorbenzoylamino)-benzoxyl]-2,3,4,5-tetrahydro-1H-benzazepin,
5-Dimethylamino-1-[2-methyl-4-(2-methylbenzoylamino)-benzoyl]-2,3,4,5-tetrahydro-1H-benzazepin,
5-Dimethylamino-1-[2-methoxy-4-(2-methylbenzoylamino)-benzoyl]-1,2,3,4-tetrahydrochinolin,
7-Chlor-5-methylamino-1-[4-(2-methylbenzoylamino)-benzoxyl]-2,3,4,5-tetrahydro-1H-benzazepin und
7-Chlor-5-methylamino-1-[4-(2-chlorbenzoylamino)-benzoxyl]-2,3,4,5-tetrahydro-1H-benzazepin ausgewählt ist, umfasst.

9. Verwendung einer Zusammensetzung nach Anspruch 8 zur Herstellung von Arzneimitteln zur Behandlung von Ventrikeldilatation und/oder Herzinsuffizienz.

## Revendications

1. Composition pharmaceutique comprenant une quantité efficace d'un inhibiteur de l'enzyme de conversion de l'angiotensine et une quantité efficace d'un antagoniste de la vasopressine de formule I 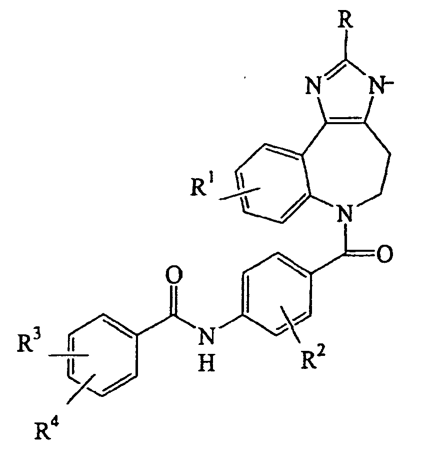 dans laquelle R et R⁵ représentent un atome d'hydrogène ou un groupe alkyle inférieur ;
R¹, R² et R³ représentent indépendamment un atome d'hydrogène, un groupe halogéno, un groupe alkyle inférieur, un groupe alkoxy inférieur, un groupe amino, un groupe alkylamino, ou un groupe dialkylamino ; et
R⁴ représente un atome d'hydrogène, un groupe phényle ou un groupe phényle substitué, et leurs sels pharmaceutiquement acceptables.

2. Composition selon la revendication 1 utilisant un inhibiteur d'ACE choisi parmi le captopril, l'énalapril, l'énalaprilat, le lisinopril, le ramipril, le zofénopril, le trandolapril, le témocapril, le ceranapril, 1' alacepril, le delapril, le pentopril, le quinapril, le quinaprilat, le moexipril, le rentiapril, le duinapril, le spirapril, le cilazapril, le perindopril et le fosinopril.

3. Composition selon la revendication 1, dans laquelle l'antagoniste de la vasopressine est le conivaptan.

4. Composition selon la revendication 2 comprenant un inhibiteur d'ACE choisi parmi l'hydrochlorure de quinapril, le ramipril, l'énalapril ou le moexipril.

5. Composition selon la revendication 4 comprenant un antagoniste de la vasopressine choisi parmi le conivaptan ou un sel de celui-ci.

6. Composition comprenant le conivaptan et le quinapril.

7. Utilisation d'une composition selon les revendications 1 à 6 pour la fabrication de produits pharmaceutiques destinés au traitement d'une dilatation ventriculaire et/ou d'une insuffisance cardiaque.

8. Composition pharmaceutique comprenant une quantité efficace d'un inhibiteur de l'enzyme de conversion de l'angiotensine et une quantité efficace d'un antagoniste de la vasopressine choisi parmi
la 5-diméthylamino-1-[4-(2-méthylbenzoylamino)-benzoyl]-2,3,4,5-tétrahydro-1H-benzazépine ;
la 5-diméthylamino-1-[2-chloro-4-(2-méthylbenzoylamino)benzoyl]-2,3,4,5-tétrahydro-1H-benzazépine ;
la 5-méthylamino-1-[2-chloro-4-(2-méthylbenzoylamino) benzoyl]-2,3,4,5-tétrahydro-1H-benzazépine ;
la 5-cyclopropylamino-1-[2-chloro-4-(2-méthylbenzoylamino) benzoxyl]-2,3,4,5-tétrahydro-1H-benzazépine ;
la 5-cyclopropylamino-1-[2-chloro-4-(2-chlorobenzoylamino) benzoxyl]-2,3,4,5-tétrahydro-1H-benzazépine ;
la 5-diméthylamino-1-[2-méthyl-4-(2-méthylbenzoylamino)benzoyl]-2,3,4,5-tétrahydro-1H-benzazépine ;
la 5-diméthylamino-1-[2-méthoxy-4-(2-méthylbenzoylamino)benzoyl]-1,2,3,4-tétrahydroquinoléine ;
la 7-chloro-5-méthylamino-1-[4-(2-méthylbenzoylamino)benzoxyl]-2,3,4,5-tétrahydro-1H-benzazépine ; et
la 7-chloro-5-méthylamino-1-[4-(2-chlorobenzoylamino)benzoxyl]-2,3,4,5-tétrahydro-1H-benzazépine.

9. Utilisation d'une composition selon la revendication 8 pour la fabrication de produits pharmaceutiques destinés au traitement d'une dilatation ventriculaire et/ou d'une insuffisance cardiaque.
